(19) Europäisches Patentamt
European Patent Office
Office européen des brevets

(11) Publication number: **0 129 239**
**B1**

(12) **EUROPEAN PATENT SPECIFICATION**

(45) Date of publication of patent specification: **11.01.89**

(21) Application number: **84106970.1**

(22) Date of filing: **18.06.84**

(51) Int. Cl.[4]: **C 01 B 33/28,** B 01 J 29/28, C 07 C 5/27, C 07 C 6/12, C 07 C 2/66, C 07 C 1/20

(54) **Method for the synthesis of ZSM-5 zeolite, at atmospheric pressure and at low temperature.**

(30) Priority: **21.06.83 IT 2169983**

(43) Date of publication of application:
**27.12.84 Bulletin 84/52**

(45) Publication of the grant of the patent:
**11.01.89 Bulletin 89/02**

(84) Designated Contracting States:
**DE FR GB IT**

(56) References cited:
**WO-A-80/02026**
**FR-A-2 217 408**
**GB-A-2 002 733**
**US-A-3 849 463**
**US-A-4 112 056**

**The file contains technical information submitted after the application was filed and not included in this specification**

(73) Proprietor: **Montedison S.p.A.**
**31, Foro Buonaparte**
**I-20121 Milan (IT)**

(72) Inventor: **Gubitosa, Giuseppe**
**5, Via della Vecchia**
**Novara (IT)**
Inventor: **Gherardi, Paola**
**14, Via Visentin**
**Novara (IT)**

(74) Representative: **Weinhold, Peter, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz**
**Siegfriedstrasse 8**
**D-8000 München 40 (DE)**

**EP 0 129 239 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The invention concerns a method for the synthesis of ZSM—5 zeolite, at atmospheric pressure and at low temperatures.

Many patents (e.g. US—A—3,702,886) deal with the synthesis of ZSM—5 zeolite, a well known catalyst for many reactions (e.g. m-xylene isomerization, toluene disproportionation, ethylbenzene synthesis etc.);

US—A—4,117,026 teaches in particular, that a ZSM—5 zeolite, having a relatively large size (average size $\geq$0.5 µm and preferably from 1 to 6 µm), can be very useful in disproportionating toluene to benzene and para-xylene. The same U.S. patent (4,117,026) states however that when relatively low temperatures are used during the zeolite preparation (e.g. 220°F, namely 104°C) said average size is rather low (0.03 µm).

It has now surprisingly been found that certain particular and original combinations of operative conditions allow to obtain, in a very easy and simple way (at atmospheric pressure and within a short time) the same ZSM—5 zeolite that was synthesized, until now, only at high temperatures (and therefore at superatmospheric pressures), or within very long periods of time (in the case of a process at atmospheric pressure) or at the price of too small a size.

The method, according to the invention, for the synthesis of ZSM—5 zeolites at atmospheric pressure and at temperatures from 60 to 100°C, comprises the following steps:

a) an aqueous solution containing a quaternary ammonium compound (QA) — e.g. tetrapropylammonium bromide [(TPA)Br] — is added to a second aqueous solution, containing a Si compound, a Na compound, and a potassium halide (KHal) according to the following molar ratios:

— $Na_2O/SiO_2 \geq 0.20$ (preferably from 0.25 to 2);

— $H_2O/SiO_2$ = from 5 to 100

— $K(Hal)/SiO_2$ = from 0.03 to 0.50;

b) a third aqueous solution, containing Al sulfate or an equivalent amount of another Al salt (e.g. Al nitrate), is added to the mixture obtained according to a);

c) to the mixture obtained according to b) an aqueous solution of $H_2SO_4$) or an equivalent amount of a monoprotic acid (e.g. $HNO_3$) or of a polyprotic acid (e.g. $H_3PO_4$) is added;

wherein the molar ratio $Y = (AQ/SiO_2)$ is from 0.02 to 0.08 or equals 0.184 or 0.368 and the molar ratio $Z = SiO_2/Al_2O_3$ is from 25 to 422, preferably from 25 to 210, said Y ratio and the molar ratio $X = OH^-/SiO_2$ being represented by points lying within triangles limited by the following 3 straight lines:

A) $Y = 0.7\ X$;

B) $X = K/Z$, wherein K = 4 (K being constant);

C) $Y = -\ 2/3\ X + 1 - H$, wherein H, constant with respect to X and Y, is obtained from the equation:

$$H = 1/2\ (45/Z)^{0.317}.$$

Alternatively, Y is from 0.02 to 0.08, Z is from 25 to 422, preferably from 25 to 210, and X is equal to 0.062.

Said (QA) quaternary compound can be replaced by a mixture of (QA) with an amine, according to the teachings of EP—A—101183.

Figure 1 graphically represents the critical triangles mentioned above, said triangles being one of the features of the invention, and figure 2 represents a narrower area, corresponding to a particularly preferred embodiment of the invention.

All the $SiO_2$ (100%) is converted into crystalline product and large outputs are possible because of the very simple apparatuses required for the synthesis; a further advantage resides in the long life-time of the zeolites prepared according to the present invention (without re-generation), especially when employed for the disproportionation of toluene or for converting methanol to propylene.

The areas of figure 1 strongly decrease[*] with the decrease of the $SiO_2/Al_2O_3$ ratio. Therefore the chances to otbain a ZSM—5 zeolite at atmospheric pressure, when said ratio is close to the lowest limit of Z, are very slim and contained within well defined and very narrow boundaries.

The present invention comprises all those equivalent embodiments that do not misrepresent the gist of the invention; e.g. the Al sulphate can be replaced by equivalent amounts of another salt (for instance nitrate) and $H_2SO_4$ can be replaced by equivalent amounts of another acid supplier of $H_3O^+$ ions, e.g. $HNO_3$ or $H_3PO_4$. The calculation of the $OH^-$ ions depends on the selected salt and acid; if the salt is Al sulphate and the acid $H_2SO_4$, the formula is (by moles):

$$X = (OH^-)/SiO_2 = 2Na_2O/SiO_2 - 2/Z—2H_2SO_4/SiO_2.$$

During the addition of the $Al^{3+}$ ions, generally in the form of sulphate or nitrate, the extension of the floculation depends on the amount of added Al. Then, also the acid (e.g. $H_2SO_4$) is added in such an amount as to obtain a more or less thick gel; best results are achieved when the amounts of acid decrease [**] with increasing amounts of Al. The crystallization time (preferably under agitation is 3 days, according to the examples, but it is possible to obtain rather good results also within shorter periods of time.

---

[*]approximately with exponential trend.

[**]within the boundaries of the figure 1 triangles.

The zeolites obtained according to the invention can be used (as such or in a modified form) in admixture with suitable amounts of oxides as binders, e.g. $SiO_2$ or $Al_2O_3$; a list of many other binders can be found e.g. in EP—A—36707. The regeneration of the catalyst can be carried out in air for a few hours at 300—600°C; a steam regeneration is described in EP—A—36704 and according to a still further method the catalyst can be regenerated by a hydrogen treatment. As to the initial activation of a zeolitic catalyst, some methods are described in EP—A—3444 and 35830; in general it is advisable to activate the catalyst for a few hours in air, at 450—750°C (preferably 540—700°C). Furthermore some conversion (e.g. methanol to propylene) exerts a stimulating effect on the zeolitic catalyst; in other words catalyst and reaction affect each other by a mutual, reciprocal and beneficial activating action.

The acid form of the zeolites (H—ZSM—5) can be very advantageously obtained when the crude product of the zeolite synthesis is dried (e.g. at 120°C), calcined (e.g. at 540°C in air for a few hours), so as to remove any residual organic compound, and then exchanged with a solution containing HCl or $NH_4NO_3$; in the second case ($NH_4NO_3$), the final acid form is obtained by means of a heat-treatment, e.g. at 400°C. Other methods for the ion exchange of the zeolites are described, for instance, in US—A—3,140,249; 3,140,251; 3,140,253 and in EP—A—68,754 and 40,276.

After the removal of the organic base and the conversion into the acid form, the zeolites prepared according to the invention show a long life time and a very high catalytic activity in many reactions, particularly in toluene disproportionation, benzene alkylation and the aromatization of olefines; they are furthermore extraordinarily active in the conversion of methanol into any kind of hydrocarbons and particularly into olefinic and aromatic hydrocarbons, especially low molecular weight olefins.

The following examples illustrate the invention.

### Example 1

22.80 g of granules of $SiO_2$ (granulated silicagel from the CARLO ERBA Company) and 10.45 g of $KF.H_2O$ were poured into a solution of 9.00 g of NaOH (98%) in 200 $cm^3$ of deionized water and the resulting suspension was kept at 100°C under stirring, until obtaining a clear solution, without any solid matter; after cooling down to room temperature, 37.19 g of (TPA) Br, dissolved in 150 $cm^3$ of deionized $H_2O$, and 2.53 g of $Al_2(SO_4)_3.18H_2O$, dissolved in 100 $cm^3$ of deionized $H_2O$, were added in this order. The thus obtained gel was heated at 80°C and drop by drop 3.00 $cm^3$ of $H_2SO_4$ (96% b.w.; density = 1.835 g/$cm^3$) were added under gentle stirring; the composition of the mixture was (by moles): $Na_2O/SiO_2 = 0.290$; $Z = SiO_2/Al_2O_3 = 100$; $Y = (TAP)/SiO_2 = 0.368$; $H_2O/SiO_2 = 65.80$; $X = OH^-/SiO_2 = 0.216$; $KF/SiO_2 = 0.292$.

The mixture was transferred to a polytetrafluoroethylene flask, equipped with cooler, stirrer and thermometer, and kept at 100°C for 3 days under gentle stirring and reflux; the product was filtered, repeatedly washed with deionized $H_2O$, until obtaining a neutral washing liquid (litmus paper) and dried at 120° for 16 hours. The solids yield was 100% and the X-ray analysis showed that the product was a ZSM—5 zeolite having a cristallinity degree of 100% (see Table 1); the composition of the product is listed in Table 1, together with the reaction conditions.

### Examples 2 and 3

Example 1 was repeated while using greater amounts of $H_2SO_4$ (respectively 4.00 and 4.50 $cm^3$), whereby the $OH^-/SiO_2$ ratio of Table 1 was modified. The results of examples 1—3 were similar.

### Example 4 (Comparative)

Example 1 was repeated, while lowering the amount of $H_2SO_4$ to 0.76 $cm^3$, whereby the value of the $OH^-/SiO_2$ ratio was 0.428, thus obtaining very poor results, as can be deduced from Table 1.

### Example 5 (Comparative)

Example 1 was repeated while bringing the $H_2SO_4$ amount up to 5.90 $cm^3$; worse results were obtained, as shown in Table 1.

### Example 6

Example 1 was repeated, while modifying the amount of Al sulphate (0.60 g) and also the amount of $H_2SO_4$ (4.65 $cm^3$); data results are given in Table 1.

### Example 7

Example 1 was repeated while increasing the amount of Al sulphate to 5.06 g and decreasing the $H_2SO_4$ amount down to 3.15 $cm^3$; data and results are given in Table 1.

### Examples 8 and 9 (Comparative)

Example 7 was repeated, while leaving the amount of Al sulphate unchanged, but adding respectively 2.00 and 3.65 $cm^3$ of $H_2SO_4$; data and results are given in Table 1.

### Example 10

Example 2 was repeated while lowering the original amount of (TPA)Br by 50%, namely to 18.60 g; data and satisfactory results are given in Table 2.

Example 11

Example 10 was repeated while adding 4.50 $cm^3$ of $H_2SO_4$; data and results are given in Table 2.

Example 12

Example 10 was repeated while doubling the amounts of $SiO_2$, NaOH, (TPA)Br, Al sulphate, potassium fluoride and $H_2SO_4$ and leaving the amount of $H_2O$ unchanged (reduction of the dilution level); data and good results are listed in Table 2.

Example 13

Example 12 was repeated, while adding 9.00 $cm^3$ of $H_2SO_4$; data and results are given in Table 2.

Example 14

Exmaple 11 was repeated, while lowering the amount of (TPA)Br to 7.68 g; data and results are given in Table 2. The average size of the crystallites was 1.5 µm.

Example 15

Example 2 was repeated, while replacing the Al-sulphate by 2.85 g of the corresponding nitrate $[Al(NO_3)_3.9H_2O]$ and replacing $H_2SO_4$ by 9.90 $cm^3$ of 65% $HNO_3$ (density = 1.400 g/$cm^3$); data and good results are given in Table 2.

Example 16

A solution of Na metasilicate, prepared by dissolving 31.02 g of NaOH in 200 $cm^3$ of deionized $H_2O$ and by adding 22.80 g of $SiO_2$ and 10.45 g of $KF.2H_2O$, was kept at 100°C until obtaining a clear solution, without any solid matter; after cooling down to room temperature, 18.60 g of (TPA)Br, dissolved in 150 $cm^3$ of deionized $H_2O$, and 2.53 g of $Al_2(SO_4)_3.18H_2O$, dissolved in 100 $cm^3$ of deionized $H_2O$, were added in this order. The gel thus obtained was heated at 80°C and 18.98 $cm^3$ of $H_2SO_4$ were added, drop by drop, under gentle stirring; the further synthesis was carried out as in Example 1. Data and results are given in Table 2.

Example 17 (Comparative)

20.27 g of $SiO_2$ granules were added to a solution of 8.00 g of NaOH in 200 $cm^3$ of deionized $H_2O$ and the resulting suspension was kept at 100°C under stirring, until obtaining a clear solution, without any solid material; after cooling down to room temperature, 33.06 g of (TPA)Br, dissolved in 100 $cm^3$ of deionized $H_2O$, and 2.53 g of $Al_2(SO_4)_3.18H_2O$, dissolved in 100 $cm^3$ of deionized $H_2O$ were added in this order. The gel thus obtained was heated to 80°C and 2.67 g of $H_2SO_4$ (96%; density = 1.835 g/$cm^3$) were added under gentle stirring; the composition of the mixture was (by moles): $Na_2O/SiO_2$ = 0.290; Z = $SiO_2/Al_2O_3$ = 100; Y = 0.368; $H_2O/SiO_2$ = 65/80; X = 0.216.

The mixture was transferred into a polytetrafluoroethylene flask and kept at 150°C for 3 days under autogenous pressure; the product was then treated as in Example 1. Data and results are given in Table 2, whereform one can take that, after 3 days, the amount of solid (completely in crystalline form) is merely 75% of the theoretical amount; a further test, for a longer period of time, has shown that the 100% solid (crystalline) yield is reached only if the heating (at 150°C and without KF) is extended for a period of 5 days.

Example 18 (Application of the zeolite catalyst)

A sample of H—ZSM5, prepared according to example 1, was calcined in air at 540°C for 16 h; 20 g of the calcined product were suspended in 400 $cm^3$ of a 0.5 M solution of $NH_4NO^3$ and kept at 80°C for 2 h under gentle stirring. Such treatment was identically repeated 5 times; eventually the product was filtered, washed until negative nitrate test (brucine test) and dried for 16 h at 120°C. The resulting $NH_4$—ZSM5 was decomposed, thereby obtaining H—ZSM5, by means of a calcination in a nitrogen stream, at 540°C for 16 h; 10 g of such H—ZSM5 were admixed with 14 $cm^3$ of a silica sol (commercially known as "Ketyensol®AKZO", containing 40% b.w. $SiO_2$), dried for 16 h at 120°C, then granulated and finally calcined for 16 h at 540°C. Thereafter 1.000 g of the fraction between 35 and 50 (Tyler) mesh was fed into a titanium ("plug flow") microreactor (360 mm long; Ø = 8 mm) and tested for the methanol conversion; operative conditions and product distribution are given in Tables 3 and 4.

Example 19

In order to ascertain that it is possible to work with more concentrated solutions, example 14 was repeated quadruplicating the amount of $SiO_2$, NaOH, (TPA)Br, potassium fluoride and Al sulphate, while adding 18.50 $cm^3$ of $H_2SO_4$ and keeping practically unaltered the water volume. Data and results are recorded in Table 5.

Example 20

Example 19 was repeated reducing to 50% the amount of (TPA)Br (15.38 g). Data and results are recorded in Table 5.

Example 21

In order to ascertain that it is possible to handle greater volumes (and outputs), example 20 was repeated multiplying by 8 all the amounts of the reactants provided for by said example 20. Data and results are recorded in Table 5.

TABLE 1

| EXAMPLE | 1 | 2 | 3 | 4(a) | 5(a) | 6 | 7 | 8(a) | 9(a) |
|---|---|---|---|---|---|---|---|---|---|
| **Reaction mixture** | | | | | | | | | |
| $Na_2O/SiO_2$ (molar) | 0.290 | see ex. 1 | see ex.1 | see ex.1 | see ex.1 | see ex.1 | see ex.1 | see ex.7 | see ex.7 |
| $SiO_2/Al_2O_3$ " | 100 | " | " | " | " | 422 | 50 | " | " |
| $(TPA)/SiO_2$ (*) " | 0.368 | " | " | " | " | see ex.1 | see ex.1 | " | " |
| $H_2O/SiO_2$ " | 65.800 | " | " | " | " | " | " | " | " |
| $KF/SiO_2$ " | 0.292 | " | " | " | " | " | " | " | " |
| $H_2SO_4$ ($cm^3$) | 3.00 | 4.00 | 4.50 | 0.76 | 5.90 | 4.65 | 3.15 | 2.00 | 3.65 |
| $OH^-/SiO_2$ (molar) | 0.216 | 0.122 | 0.074 | 0.428 | −0.058(d) | 0.121 | 0.122 | 0.231 | 0.075 |
| **End product** | | | | | | | | | |
| Solid yield | 100% | 100% | 100% | 23% | 84% | 100% | 100% | 70% | 90% |
| ZSM-5 phase (c) | 100% | 100% | 100% | 10%(b) | 0% | 100% | 100% | 30%(b) | 65%(b) |

(a) comparative; (b) + amorphous; (c) with respect to the solid; (d) the minus sign has no physical meaning but comes merely from the particular kind of calculation; (*) moles of (TPA)Br per mole of $SiO_2$.

TABLE 2

| EXAMPLE | 10 | 11 | 12 | 13 | 14 | 15 | 16 | 17 (a) |
|---|---|---|---|---|---|---|---|---|
| $Na_2O/SiO_2$ (molar) | see ex.2 | see ex.10 | see ex.10 | see ex.12 | see ex.11 | 0.290 | 1.00 | 0.290 |
| $SiO_2/Al_2O_3$ " | " | " | " | " | 100 | 100 | 100 | 100 |
| $(TPA)/SiO_2$ " (*) | 0.184 | " | " | " | 0.076 | 0.368 | 0.184 | 0.368 |
| $H_2O/SiO_2$ " | see ex.2 | see ex.10 | 32,900 | " | 65.800 | 65.899 | 65.800 | 65.800 |
| $KF/SiO_2$ " | " | " | 0.292 | " | 0.292 | 0.292 | 0.292 | - |
| $H_2SO_4$ or $HNO_3$ ($cm^3$) | see ex.2 | 4.50 ($H_2SO_4$) | 8.00 ($H_2SO_4$) | 9.00 ($H_2SO_4$) | see ex.11 | 9.90 ($HNO_3$) | 18.98 ($H_2SO_4$) | 2.67 ($H_2SO_4$) |
| $OH^-/SiO_2$ (molar) | 0.122 | 0.074 | 0.122 | 0.074 | 0.074 | 0.124 | 0.124 | 0.216 |
| End product | | | | | | | | |
| Solid yield | 100% | 100% | 100% | 100% | 100% | 100% | 100% | 75% |
| ZSM-5 phase (b) | 100% | 100% | 100% | 100% | 100% | 100% | 100% | 100% |

(a) comparative; (b) with respect to the solid;

(*) moles of (TPA)Br per mole of $SiO_2$.

TABLE 3

OPERATIVE CONDITIONS AND PRODUCT DISTRIBUTION

| Operative conditions | Products | % b.w. (***) |
|---|---|---|
| Temperature: 400°C | DME (**): | - |
| LHSV (*): 2 $h^{-1}$ | $C_2H_4$: | 4.9 |
| $CH_3OH$ conversion: 95% | $C_3H_6$: | 39.2 |
| Time of the run: 50 h | liquid fraction (hydrocarbons): | 55.9 |

TABLE 4

DISTRIBUTION OF THE PRODUCTS WITHIN THE LIQUID (HYDROCARBONS) FRACTION (AT 25°C)

| | % b.w. | PM (average molecular weight) |
|---|---|---|
| $C_5$ | 3.7 | 70 |
| $C_6$ | 38.5 | 84 |
| $C_7$ | 12.0 | 98 |
| $C_8$ | 8.8 | 112 |
| $C_9$ | 19.5 | 126 |
| $C_{10}$ | 13.5 | 140 |
| $C_{11}$ | 3.2 | 156 |

---

(*) liters/h of liquid feed per liter of (bound) zeolite;

(**) dimethyl ether;

(***) the calculation does not take into account $H_2O$

TABLE 5

| EXAMPLES | 19 | 20 | 21(***) |
|---|---|---|---|
| Reaction mixture | | | |
| $Na_2O/SiO_2$ (molar) | see ex.14 | see ex.19 | see ex.20 |
| $SiO_2/Al_2O_3$ " | 100 | 100 | " |
| $(TPA)/SiO_2$ " (*) | 0.076 | 0.038 | " |
| $H_2O/SiO_2$ " | 16.400 | s.ex.19 | " |
| $KF/SiO_2$ " | 0.292 | s. ex.19 | " |
| $H_2SO_4$ ($cm^3$) | 18.50 | s.ex.19 | " |
| $OH^-/SiO_2$ (molar) | 0.062 | s.ex.19 | " |
| data survey after | 3 days | 3 days | 3 days |
| End product: | | | |
| Solid yield | 100% | 100% | 100% |
| ZSM-5 phase (**) | 100% | >90% | 100% |

(*) moles of (TPA)Br per mole of $SiO_2$.

(**) with respect to the solid.

(***) greater amount of reactants (8 times with respect to example 20).

## Claims

1. A method for the synthesis of ZSM—5 zeolites at atmospheric pressure and at a temperature of from 60 to 100°C, wherein:

a) an aqueous solution containing a quaternary ammonium compound (QA) is added to a second aqueous solution, containing a Si compound, a Na compound, and a potassium halide (KHal) according to the following molar ratios:

— $Na_2O/SiO_2 \geq 0.20$ (preferably from 0.25 to 2);

— $H_2O/SiO_2$ = from 5 to 100;

— $K(Hal)/SiO_2$ = from 0.03 to 0.50;

b) a third aqueous solution, containing Al sulfate or an equivalent amount of another Al salt (e.g. Al nitrate), is added to the mixture obtained according to a);

c) to the mixture obtained according to b) an aqueous solution of $H_2SO_4$ or an equivalent amount of a monoprotic acid (e.g. $HNO_3$) or of a polyprotic acid (e.g. $H_3PO_4$) is added;

and wherein the molar ratio $Y = (QA/SiO_2)$ is from 0.02 to 0.08 and the molar ratio $Z = SiO_2/Al_2O_3$ is from 25 to 422, said Y ratio and the molar ratio $X = OH^-/SiO_2$ being represented by points lying within triangles limited by the following 3 straight lines:

A) $Y = 0.7\ X$;

B) $X = K/Z$, wherein $K = 4$ (K being constant);

C) $Y = -2/3\ X + 1 - H$, wherein the constant is obtained from the equation: $H = 1/2\ (45/Z)^{0.317}$.

2. A method for the synthesis of ZSM—5 zeolites at atmospheric pressure and at a temperature of from 60 to 100°C, wherein:

a) an aqueous solution containing a quaternary ammonium compound (QA) is added to a second aqueous solution, containing a Si compound, a Na compound, and a potassium halide (KHal), according to the molar ratios:

— $Na_2O/SiO_2 \geqslant 0.20$ (preferably from 0.25 to 2);

— $H_2O/SiO_2$ = from 5 to 100;

— $K(Hal)/SiO_2$ = from 0.03 to 0.50;

b) a third aqueous solution, containing Al sulfate or an equivalent amount of another Al salt (e.g. Al nitrate), is added to the mixture obtained according to a);

c) to the mixture obtained according to b) an aqueous solution of $H_2SO_4$) or an equivalent amount of a monoprotic acid (e.g. $HNO_3$) or of a polyprotic acid (e.g. $H_3PO_4$) is added;

and wherein the molar ratio $Y = (QA/SiO_2)$ is 0.368 and the molar ratio $Z = SiO_2/Al_2O_3$ is from 25 to 422, said Y ratio and the molar ratio $X = OH^-/SiO_2$ being represented by points lying within triangles limited by the following 3 straight lines:

A) $Y = 0.7\ X$;

B) $X = K/Z$; wherein K = 4 (K being constant);

C) $Y = -2/3\ X + 1 - H$, wherein the constant is obtained from the equation: $H = 1/2\ (45/Z)^{0.317}$.

3. A method for the synthesis of ZSM—5 zeolites at atmospheric pressure and at a temperature of from 60 to 100°C, wherein:

a) an aqueous solution containing a quaternary ammonium compound (QA) is added to a second aqueous solution, containing a Si compound, a Na compound, and a potassium halide (KHal) according to the following molar ratios:

— $Na_2O/SiO_2 \geqslant 0.20$ (preferably from 0.25 to 2);

— $H_2O/SiO_2$ = from 5 to 100;

— $K(Hal)/SiO_2$ = from 0.03 to 0.50;

b) a third aqueous solution, containing Al sulfate or an equivalent amount of another Al salt (e.g. Al nitrate), is added to the mixture obtained according to a);

c) to the mixture obtained according to b) an aqueous solution of $H_2SO_4$) or an equivalent amount of a monoprotic acid (e.g. $HNO_3$) or of a polyprotic acid (e.g. $H_3PO_4$ is added;

and wherein the molar ratio $Y = (QA/SiO_2)$ is 0.184 and the molar ratio $Z = SiO_2/Al_2O_3$ is from 25 to 422, said Y ratio and the molar ratio $X = OH^-/SiO_2$ being represented by points lying within triangles limited by the following 3 straight lines:

A) $Y = 0.7\ X$;

B) $X = K/Z$, wherein K = 4 (K being constant);

C) $Y = -2/3\ X + 1 - H$, wherein the constant is obtained from the equation: $H = 1/2\ (45/Z)^{0.317}$.

4. A method for the synthesis of ZSM—5 zeolites at atmospheric pressure and at a temperature of from 60 to 100°C, wherein:

a) an aqueous solution containing a quaternary ammonium compound (QA) is added to a second aqueous solution, containing a Si compound, a Na compound, and a potassium halide (KHal) according to the following molar ratios:

— $Na_2O/SiO_2 \geqslant 0.20$ (preferably from 0.25 to 2);

— $H_2O/SiO_2$ = from 5 to 100;

— $K(Hal)/SiO_2$ = from 0.03 to 0.50;

b) a third aqueous solution, containing Al sulfate or an equivalent amount of another Al salt (e.g. Al nitrate), is added to the mixture obtained according to a);

c) to the mixture obtained according to b) an aqueous solution of $H_2SO_4$ or an equivalent amount of a monoprotic acid (e.g. $HNO_3$) or of a polyprotic acid (e.g. $H_3PO_4$) is added;

and wherein the molar ratio $Y = (QA/SiO_2)$ is from 0.02 to 0.08 and the molar ratio $Z = SiO_2/Al_2O_3$ is from 25 to 422, and the molar ratio $X = OH^-/SiO_2$ being equal to 0.062.

5. A method according to any of claims 1 to 4 wherein Z is from 25 to 210.

6. A method according to any of claims 1 to 5, wherein the total dilution namely the "total $H_2O$"/$SiO_2$ molar ratio, is from 7 to 120 (preferably from 15 to 80).

7. A method according to any of claims 1 to 5, wherein said potassium halide is selected from the group comprising potassium chloride and potassium fluoride.

8. Use of the zeolites, obtained according to the method of claims 1 to 7, for an acid-catalyzed reaction, in particular for the xylene isomerization, the toluene disproportionation and the benzene alkylation.

9. Use of the zeolites, obtained according to the method of claims 1 to 7, for the methanol conversion to light olefines and in particular to propylene.

**Patentansprüche**

1. Verfahren zur Herstellung von ZSM—5—Zeoliten bei atmosphärischem Druck und bei einer Temperatur von 60 bis 100°C, bei welchem

a) eine wässrige Lösung, die eine quaternäre Ammoniumverbindung (QA) enthält, zu einer zweiten wassrigen Lösung, die eine Si-Verbindung, eine Na-Verbindung und ein Kaliumhalogenid (KHal) entsprechend den molaren Verhältnissen

— $Na_2O/SiO_2$ ⩾0,20 (vorzugsweise von 0,25 bis 2),
— $H_2O/SiO_4$ = von 5 bis 100
— $KHal/SiO_2$ = von 0,03 bis 0,50

enthält, zugeführt wird,

b) eine dritte wässrige Lösung, die Al-Sulfat oder eine äquivalente Menge eines anderen Al-Salzes (z.B. Al-Nitrat) ethält, zu der gemäß a) erhaltenen Mischung zugefügt wird,

c) zu der gemäß b) erhaltenen Mischung eine wässrige Lösung von $H_2SO_4$ oder einer äquivalenten Menge einer monoprotischen Säure (z.B. $HNO_3$) oder einer polyprotischen Säure (z.B. $H_3PO_4$) zugefügt wird,

und bei welchem das molare Verhältnis Y = ($QA/SiO_2$) von 0,02 bis 0,08 liegt und das molare Verhältnis Z = $SiO_2/Al_2O_3$ von 25 bis 422 liegt, wobei das Y—Verhältnis und das molare Verhältnis X = $OH^-/SiO_2$ dargestellt wird durch Punkte, die innerhalb von Dreiecken liegen, die durch die folgenden drei geraden Linien begrenzt werden:

A) Y = 0,7 X,
B) X = K/Z, worin K = 4 ist (und K konstant ist),
C) Y = −2/3 X + 1 − H, worin die Konstante H erhalten wird aus der Gleichung: $H = 1/2\ (45/Z)^{0,317}$.

2. Verfahren zur Herstellung von ZSM—5—Zeoliten bei atmosphärischem Druck und bei einer Temperatur von 60 bis 100°C, bei welchem

a) eine wässrige Lösung, enthaltend eine quaternäre Ammoniumverbindung (QA), enthaltend zu einer zweiten wässrigen Lösung, eine Si-Verbindung, eine Na-Verbindung und ein Kaliumhalogenid (KHal) entsprechend den molaren Verhältnissen:

— $Na_2O/SiO_2$ ⩾0,20 (vorzugsweise von 0,25 bis 2),
— $H_2O/SiO_2$ = von 5 bis 100,
— $KHal/SiO_2$ = von 0,03 bis 0,50,

zugeführt wird,

b) eine dritte wässrige Lösung, enthaltend Al-Sulfat oder eine äquivalente Menge eines anderen Al-Salzes (z.B. Al-Nitrat), zu der gemäß a) erhaltenen Mischung zugefügt wird,

c) zu der gemäß a) erhaltenen Mischung eine wässrige Lösung von $H_2SO_4$ oder einer äquivalenten Menge einer Monoprotischen Säure (z.B. $HNO_3$) oder einer polyprotischen Säuren (z.B. $H_2PO_4$) zugefügt wird,

und bei welchem das molare Verhältnis Y = ($QA/SiO_2$) 0,368 beträgt und das molare Verhältnis Z = $SiO_2/Al_2O_3$ von 25 bis 422 betragt, wobei das Y—Verhältnis und das molare Verhältnis X = $OH^-/SiO_2$ durch Punkte dargestellt wird, die innerhalb von Dreiecken liegen, die durch die folgenden drei geraden Linien begrenzt werden:

A) Y = 0,7 X,
B) X = K/Z, wobei K = 4 ist (und K konstant ist),
C) Y = −2/3 X + 1 − H, worin die Konstante H erhalten wird aus der Gleichung: $H = 1/2\ (45/Z)^{0,317}$.

3. Verfahren zur Herstellung von ZSM—5—Zeoliten bei atmosphärischem Druck und bei einer Temperatur von 60 bis 100°C, bei welchem

a) eine wässrige Lösung, enthaltend eine quaternäre Ammoniumverbindung (QA), zu einer zweiten wässrigen Lösung, enthaltend eine Si-Verbindung, eine Na-Verbindung und ein Kaliumhalogenid (KHal) gemäß den molaren Verhältnissen

— $Na_2O/SiO_2$ ⩾0,20 (vorzugsweise von 0,25 bis 2),
— $H_2O/SiO_2$ = von 5 bis 100,
— $KHal/SiO_2$ = von 0,03 bis 0,50,

zugeführt wird,

b) eine dritte wässrige Lösung, enthaltend Al-Sulfat oder eine äquivalente Menge eines anderen Al-Salzes (z.B. Al-Nitrat), zu der gemäß a) erhaltenen Mischung zugefügt wird,

c) zu der gemäß b) erhaltenen Mischung eine wässrige Lösung von $H_2SO_4$ oder einer äquivalenten Menge einer Monoprotischen Säure (z.B. $HNO_3$) oder einer polyprotischen Säure (z.B. $H_2PO_4$) zugefügt wird,

und bei welchem das molare Verhältnis X = ($QA/SiO_2$) 0,184 beträgt und das molare Verhältnis Z = $SiO_2/Al_2O_3$ von 25 bis 422 beträgt, wobei das Y—Verhältnis und das molare Verhältnis X = $OH^-/SiO_2$ durch Punkte dargestellt wird, die innerhalb von Dreiecken liegen, die durch die folgenden drei geraden Linien bengrenzt werden:

A) Y = 0,7 X,
B) X = K/Z, worin K = 4 ist (und K konstant ist),
C) Y = −2/3 X + 1 − H, worin die Konstante H erhalten wird aus der Gleichung: $H = 1/2\ (45/Z)^{0,317}$.

4. Verfahren zur Herstellung von ZSM—5—Zeoliten bei atmosphärischem Druck und bei einer Temperatur von 60 bis 100°C, bei welchem

a) eine wässrige Lösung, enthaltend eine quaternäre Ammoniumverbindung (QA), zu einer zweiten wassrigen Lösung, enthaltend eine Si-Verbindung, eine Na-Verbindung und ein Kaliumhalogenid (KHal) gemäß den molaren Verhältnissen

— $Na_2O/SiO_2 \geq 0{,}20$ (vorzugsweise von 0,25 bis 2),

— $H_2O/SiO_4$ = von 5 bis 100,

— $KHal/SiO_2$ = von 0,03 bis 0,50,

zugeführt wird,

b) eine dritte wässrige Lösung, enthaltend Al-Sulfat oder eine äquivalente Menge eines anderen Al-Salzes (z.B. Al-Nitrat), zu der gemäß a) erhaltenen Mischung zugefügt wird,

c) zu der gemäß b) erhaltenen Mischung eine wässrige Lösung von $H_2SO_4$ oder einer äquivalenten Menge einer monoprotischen Säure (z.B. $HNO_3$) oder einer polyprotischen Säure (z.B. $H_2PO_4$) zugefügt wird,

und bei welchem das molare Verhältnis Y = ($QA/SiO_2$) 0,02 bis 0,08 beträgt und das molare Verhältnis Z = $SiO_2/Al_2O_3$ von 25 bis 422 beträgt und das molare Verhältnis X = $OH^-/SiO_2$ gleich 0,062 ist.

5. Verfahren nach irgendeinem der Ansprüche 1 bis 4, worin Z von 25 bis 210 ist.

6. Verfahren nach irgendeinem der Ansprüche 1 bis 5, worin die gesamte Verdünnung, nämlich das molare Verhältnis von "gesamtem $H_2O$"/$SiO_2$, von 7 bis 120 (vorzugsweise von 15 bis 80) beträgt.

7. Verfahren nach irgendeinem der Ansprüche 1 bis 5, worin das Kaliumhalogenid ausgewählt ist aus der Gruppe, die Kaliumchlorid und Kaliumfluorid umfaßt.

8. Die Verwendung von Zeoliten, erhalten nach dem Verfahren der Ansprüche 1 bis 7, für eine säurekatalysierte Reaktion, insbesondere zur Xylolisomerisation, zur Toluoldisproportionierung und zur Benzolalkylierung.

9. Die Verwendung von Zeoliten, erhalten nach dem Verfahren der Ansprüche 1 bis 7, zur Methanolumwandlung in leichte Olefine und insbesondere in Propylen.

**Revendications**

1. Un procédé de synthèse de zéolite ZSM—5 à pression atmosphérique et à une température comprise entre 60 et 100°C, dans lequel:

a) une solution aqueuse contenant un composé d'ammonium quaternaire (QA) est ajoutée à une deuxième solution aqueuse contenant un composé de silicium, un dérivé de sodium et un halogénure de potassium (KHal), utilisés selon les rapports molaires:

— $Na_2O/SiO_2 \geq 0{,}20$ (de préference compris entre 0,25 et 2);

— $H_2O/SiO_2$ compris entre 5 et 100;

— $KHal/SiO_2$ compris entre 0,03 et 0,50;

b) une troisième solution aqueuse contenant du sulfate d'aluminium ou une quantité équivalente d'un autre sel d'aluminium (par example nitrate d'aluminium), est ajoutée au mélange obtenu dans l'étape a);

c) au mélange obtenu dans l'étape b), est ajoutée une solution aqueuse de $H_2SO_4$ ou une quantité équivalente d'un acide monoprotique (par exemple $HNO_3$) ou d'un acide polyprotique (par exemple $H_3PO_4$;

et dans lequel le rapport molaire Y = ($QA/SiO_2$) est compris entre 0,02 et 0,08 et le rapport molaire Z = $SiO_2/Al_2O_3$ est compris entre 25 et 422, ce rapport Y et le rapport molaire X = $OH^-/SiO_2$ étant représentés par des points se trouvant dans des triangles limités par les trois lignes droites suivantes:

A) $Y = 0{,}7X$;

B) $X = K/Z$, dans lequel K = 4 (K étant une constante);

C) $Y = -2/3\,X + 1 - H$, dans lequel la constante H est obtenue à partir de l'équation: $H = 1/2\,(45/Z)^{0{,}317}$.

2. Un procédé de synthèse de zéolite ZSM—5 à pression atmosphérique et à une température comprise entre 60 et 100°C, dans lequel:

a) une solution aqueuse contenant un composé d'ammonium quaternaire (QA) est ajoutée à une deuxième solution aqueuse contenant un composé de silicium, un dérivé de sodium et un composé de potassium (KHal), selon les rapports molaires:

— $Na_2O/SiO_2 \geq 0{,}20$ (de préference de 0,25 et 2);

— $H_2O/SiO_2$ compris entre 5 et 100;

— $KHal/SiO_2$ compris entre 0,03 et 0,50;

b) une troisième solution aqueuse contenant du sulfate d'aluminium ou une quantité équivalente d'un autre sel d'aluminium (par exemple le nitrate d'aluminium) est ajoutée au mélange obtenu dans l'étape a);

c) au mélange obtenu dans l'étape b), on ajoute une solution aqueuse de $H_2SO_4$ ou une quantité équivalente d'un acide monoprotique (par exemple $HNO_3$) ou d'un acide polyprotique ($H_3PO_4$),

et dans lequel le rapport molaire Y = ($QA/SiO_2$) est égal à 0,368 et le rapport molaire Z = $SiO_2/Al_2O_3$ est compris entre 25 et 422, ce rapport Y et le rapport molaire X = $OH^-/SiO_2$ étant représentés par des points se trouvant dans les triangles limités par les trois lignes droites suivantes:

A) $Y = 0{,}7X$;

B) $X = K/Z$, dans lequel K = 4 (K étant une constante);

C) $Y = -2/3\,X + 1 - H$, dans lequel la constante H est obtenue à partir de l'équation: $H = 1/2\,(45/Z)^{0{,}317}$.

3. Un procédé de synthèse de zéolite ZSM—5 à pression atmosphérique et à une température comprise entre 60 et 100°C, dans lequel:

a) une solution aqueuse contenant un composé d'ammonium quaternaire (QA) est ajoutée à une deuxième solution aqueuse contenant un composé de silicium, un dérivé de sodium et un composé de potassium (KHal), selon les rapports molaires:

— $Na_2O/SiO_2 \geq 0,20$ (de préférence de 0,25 à 2);
— $H_2O/SiO_2$ de 5 à 100;
— $KHal/SiO_2$ compris entre 0,03 et 0,50;

b) une troisième solution aqueuse contenant du sulfate d'aluminium ou une quantité équivalente d'un autre sel d'aluminium (par exemple le nitrate d'aluminium) est ajoutée au mélange obtenu dans l'étape a);

c) au mélange obtenu dans l'étape b), ou ajoute une solution aqueuse de $H_2SO_4$ ou une quantité équivalente d'un acide monoprotique (par exemple $HNO_3$) ou d'un acide polyprotique ($H_3PO_4$),

et dans lequel le rapport molaire $Y = (QA/SiO_2)$ est égal à 0,184 et le rapport molaire $Z = SiO_2/Al_2O_3$ est compris entre 25 et 422, ce rapport Y et le rapport molaire $X = OH^-/SiO_2$ étant représentés par des points se trouvant dans des triangles limités par les trois lignes droites suivantes:

A) $Y = 0,7X$;
B) $X = K/Z$, dans lequel K = 4 (K étant une constante);
C) $Y = -2/3\,X + 1 - H$, dans lequel la constante H est obtenue à partir de l'équation: $H = 1/2\,(45/Z)^{0,317}$.

4. Un procédé de synthèse de zéolite ZSM—5 à pression atmosphérique et à une température comprise entre 60 et 100°C, dans lequel:

a) une solution aqueuse contenant un composé d'ammonium quaternaire (QA) est ajoutée à une deuxième solution aqueuse contenant un composé de silicium, un dérivé de sodium et un composé de potassium (KHal), selon les rapports molaires:

— $Na_2O/SiO_2 \geq 0,20$ (de préférence de 0,25 et 2);
— $H_2O/SiO_2$ compris entre 5 et 100;
— $KHal/SiO_2$ compris entre 0,03 et 0,50;

b) une troisième solution aqueuse contenant du sulfate d'aluminium ou une quantité équivalente d'un autre sel d'aluminium (par exemple le nitrate d'aluminium) est ajoutée au mélange obtenu dans l'étape a);

c) au mélange obtenu dans l'étape b), on ajoute une solution aqueuse de $H_2SO_4$ ou une quantité équivalente d'un acide monoprotique (par exemple $HNO_3$) ou d'un acide polyprotique ($H_3PO_4$),

et dans lequel le rapport molaire $Y = (QA/SiO_2)$ est compris entre 0,02 et 0,08, le rapport molaire $Z = SiO_2/Al_2O_3$ est compris entre 25 et 422, et le rapport molaire $X = OH^-/SiO_2$ étant égal à 0,062.

5. Un procédé selon l'une quelconque des revendications 1 à 4, dans lequel Z est compris entre 25 et 210.

6. Un procédé selon l'une quelconque des revendications 1 à 5, dans lequel la dilution totale, à savoir le rapport molaire "$H_2O$ totale"/$SiO_2$ est compris entre 7 et 120 (de préférence entre 15 et 80).

7. Un procédé selon l'une quelconque des revendications 1 à 5, dans lequel ledit halogénure de potassium est choisi dans le groupe comprenant le chlorure de potassium et le fluorure de potassium.

8. Utilisation des zéolites obtenues selon le procédé des revendications 1 à 7, pur la réaction catalysée par acide, en particulier pour l'isomérisation du xylène, la disproportionnation du toluène et l'alkylation du benzène.

9. Utilisation des zéolites obtenues selon le procédé des revendications 1 à 7, pour la conversion du méthanol en oléfines légères et, en particulier, en propylène.

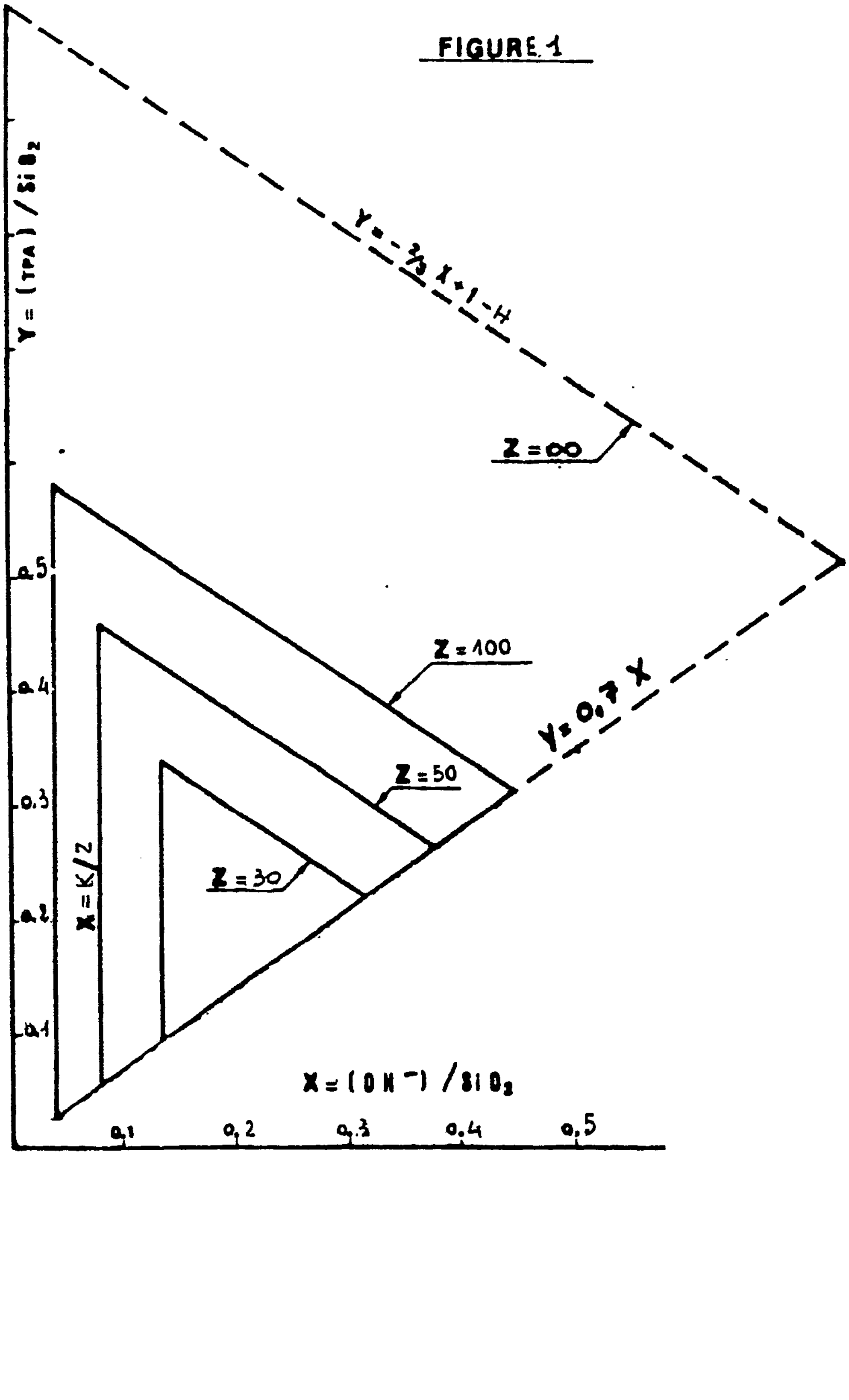
FIGURE 1
Y = (TPA) / Si O2
Y = -2/3 X + 1-4
Z = ∞
Z = 100
Z = 50
Z = 30
Y = 0,7 X
X = K/Z
X = (OH⁻) / Si O2
0.1
0.2
0.3
0.4
0.5
0,1
0,2
0,3
0,4
0,5

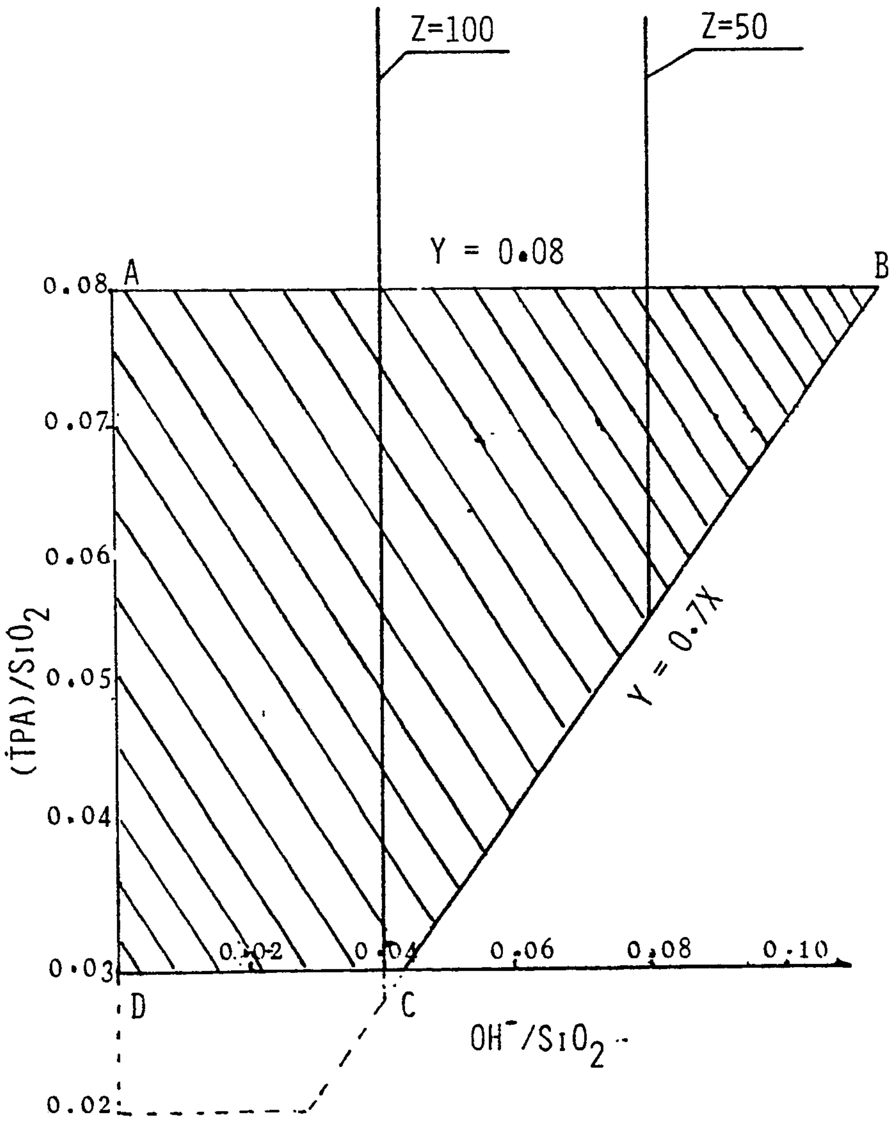
Z=100
Z=50
Y = 0.08
A
B
0.08
0.07
0.06
0.05
0.04
0.03
0.02
(TPA)/SiO2
Y = 0.7X
0.02
0.04
0.06
0.08
0.10
D
C
OH⁻/SiO2

FIGURE 2